# EUROPEAN PATENT SPECIFICATION

(11) **EP 3 836 789 B1**
(45) Date of publication and mention of the grant of the patent: **23.10.2024**
(21) Application number: 20764176.2
(22) Date of filing: 09.07.2020
(51) Int. Cl.: A01N 63/30, A01N 25/00, A01N 25/08, A01N 25/12, A01N 25/22, A01N 25/34, A01P 7/00, C12N 1/14

(54) **BIOLOGICAL PREPARATION FOR PLANT PROTECTION, METHOD FOR ITS PREPARATION AND METHOD OF ITS USE**
BIOLOGISCHES PRÄPARAT ZUM PFLANZENSCHUTZ, VERFAHREN ZU SEINER HERSTELLUNG UND SEINE VERWENDUNG
PRÉPARATION BIOLOGIQUE DE PROTECTION DES PLANTES, SON PROCÉDÉ DE PRÉPARATION ET SON PROCÉDÉ D'UTILISATION

(30) Priority: 12.07.2019 SK PP792019
(43) Date of publication of application: 23.06.2021
(73) Proprietor: Národné Lesnícke Centrum, 960 01 Zvolen (SK)
(72) Inventor: KUNCA, Andrej, 969 01 Banská Stiavnica (SK); LALÍK, Michal, 966 15 Banská Belá (SK); GALKO, Juraj, 969 01 Banská Stiavnica (SK)
(74) Representative: Litváková a spol., s.r.o.
(86) International application number: PCT/SK2020/050007
(87) International publication number: WO 2021/010905

(56) References cited:
- CN-A- 101 843 196
- CN-A- 103 058 721
- US-A- 5 759 562
- GOLA DEEPAK ET AL: "Production and shelf life evaluation of three different formulations of Beauveria bassiana in terms of multimetal removal", BIOTECHNOLOGY RESEARCH AND INNOVATION, vol. 3, no. 2, 1 July 2019 (2019-07-01), pages 242 - 251, XP055834156, ISSN: 2452-0721, Retrieved from the Internet <URL:https://doi.org/10.1016/j.biori.2019.06.001> DOI: 10.1016/j.biori.2019.06.001
- TUAN ANH PHAM ET AL: "Production of Blastospore of Entomopathogenic Beauveria bassiana in a Submerged Batch Culture", MYCOBIOLOGY, 30 September 2009 (2009-09-30), pages 218 - 224, XP055445092, Retrieved from the Internet <URL:https://www.ncbi.nlm.nih.gov/pmc/articles/PMC3749392/pdf/mb-37-218.pdf> [retrieved on 20180126]
- RAVNEEL KUMAR ET AL: "Mango Pest Survey View project", JOURNAL OF SOUTH PACIFIC AGRICULTURE, vol. 20, 1 January 2017 (2017-01-01), pages 44 - 52, XP055739063
- "Microbial Control of Insect and Mite Pests", 1 January 2017, ELSEVIER, ISBN: 978-0-12-803527-6, article S.T. JARONSKI ET AL: "Mass Production of Fungal Entomopathogens", pages: 141 - 155, XP055738956, DOI: 10.1016/B978-0-12-803527-6.00009-3
- STEVEN EDGINGTON2 ET AL: "Photoprotection of Beauveria bassiana: testing simple formulations for control of the coffee berry borer", INTERNATIONAL JOURNAL OF PEST MANAGEMENT, vol. 46, no. 3, 1 January 2000 (2000-01-01), pages 169 - 176, XP055739668
- SIAMAK ROSHANDEL ET AL: "The effects of natural substrates on the sporulation and viability of conidia and blastospores of Metarhizium anisopliae", BIOCONTROL IN PLANT PROTECTION, vol. 4, no. 2016, 1 December 2016 (2016-12-01), pages 94 - 104, XP055739788

## Description

### Technical Field

The present invention relates to a biological preparation for plant protection, to a method of its preparation and to a method of its use, and belongs to the field of the environment, agriculture, forestry and forest management. The invention belongs in particular to the field of plant protection from pests.

### Background Art

Chemicals, so-called pesticides, are most often used for plant protection from pests. In terms of environmental protection and occupational safety is their use problematic. Moreover, pests become more and more resistant to these chemicals and thus reduce or even lose their effectiveness against these pests. The application of pesticides is usually carried out by diluting the concentrate with water and applying it to the surface of the plants by spraying, thus forming a thin film on the surface, which also ensures the protection of the plants for several weeks.

Biological plant protection, which is more ecological and can be used even in an environment where it is not possible to use chemical preparations, e.g. for hygienic or ecological reasons, as biological plant protection products are effective for target organisms and are harmless to non-target organisms, including humans, comes increasingly to the fore.

One method of biological protection of plants from pests is using spores of entomopathogenic fungi, which infect the pest and cause its death.

Slovak Patent Document No. 288591 describes a strain of entomopathogenic fungus *Beauveria bassiana*, which is highly virulent for images of the European spruce bark beetle and aqueous suspension of fungal conidia is applied to surfaces of infested trees or to traps.

When applying the spore suspension using the regular method of pesticide application, i.e. diluting the suspension and applying it onto the plant's surface, the entomopathogenic fungus is exposed to adverse conditions, mainly UV radiation and drought. In such adverse conditions, the mycelium of the entomopathogenic fungus is inactivated, thereby limiting the effectiveness of the plant's anti-pest protection to a maximum of 1 week.

In forestry, biological protection is most often used against insect pests by applying a solution of *Bacillus thuringiensis* bacteria spores against overgrown butterfly caterpillars on deciduous tree leaves or by applying the entomopathogenic fungus *Beauveria bassiana* in the form of a powder or suspension. In the case of beetles (e. g. large pine weevil, European spruce bark beetle), whose larvae live under the bark and beetles live scattered in the growth, the application of the biological preparation in the same way as to leaf-eating insect larvae is ineffective. By spraying the wood surface, the spores of the fungus do not get under the bark at all, and such overgrowth would take several weeks through the pits.

The solution to these problems is to find alternative ways of applying spores. Various liquid or solid carriers are disclosed in the patent applications in order to prolong the duration of action of entomopathogens.

International patent application PCT/NZ2009/000217 describes the use of a strain of the entomopathogenic fungus *Beauveria bassiana* as a biological control agent for pathogenic insects, wherein the biological control is also performed by using an agriculturally acceptable liquid or solid carrier.

Czech patent document no. 300 701 describes use of a strain of the entomopathogenic fungus *Isaria fumosorosea* in pest and tick elimination. The elimination method is based on dispersing the entomopathogenic fungal spores in liquid or a solid inert carrier. The invention does not deal with life time of the product and, therefore, neither with its effectiveness in natural conditions, affected by UV radiation and drought. When applying the spore suspension using a method used to apply pesticides, the protection of plants from pests is limited to a short period of time.

Russian patent No. 2 308 191 describes a composition used for biological protection of plants that includes hard carrier containing wheat grains and microbiological source of lysine; the composition further includes succinic acid and a mixed culture of entomopathogenic fungi. The carrier is based on wheat grains. The invention does not deal with life time of the product and, therefore, neither its effectiveness in natural conditions, affected by UV radiation and drought. When spores of entomopathogenic fungi are applied on wheat grains, the exposure time and, therefore, effectiveness of the preparation is very short.

United States patent application US 2006/011066 describes a method of selective application of entomopathogenic fungi, using a device for contaminant attraction, where the fungal spores are attached to absorption material with gel reagent or other additives applied in order to maintain appropriate humidity, facilitating spore survival. Patent application No. US 5 759 562 A discloses improved compositions for controlling soil pests, particularly insect pests, said composition comprising a biological control agent. Spheric granules have diameter of 0.5 mm and comprise clay, nutrient source and *Beauveria bassiana* at the ratio 50:33:17. Nutrient source can be ground buckwheat, ground sago, ground aduki beans, ground barley flakes, ground chick peas, ground sunflower seed, ground almond, brown rice flour, carob, fine wheat bran, whole egg replacer and whole wheat semolina.

The invention described in CN 101 843 196 A belongs to the technical field of fungus culture, particularly to a method for culturing mycelia and sporocarps of aweto, which solves the problem that artificial aweto in the prior art can not be cultured in a large scale. The method comprises the following steps of: selecting *Cephalosporium acremonium* and *Isaria cicadae*; preparing a *Cephalosporium acremonium* liquid strain and an *Isaria cicadae* liquid strain; preparing a solid culture medium from corn grit, soybean flour, wheat grains, sorghum grains, silkworm chrysalis meal, whole chicken eggs, black bean coarse powder containing hulls, degreased linseed oil and a nutrient solution, wherein the nutrient solution comprises chicken blood, pine needles, carrots, black beans and cattle bone; evenly mixing, and inoculating; and culturing to obtain the sporocarps and the mycelia. The detection indicates that the sporocarps contain at least 0.02% of adenosine and at least 0.1% of cordycepin, and the mycelia contain at least 0.055% of adenosine; the finger-print test result indicates that the degree of similarity of the sporophores and the mycelia is greater than 97%; and acute toxicity experiments and long-term toxicity experiments indicate that the sporophores and the mycelia have no toxicity and can be used as substitutes of natural aweto.

The invention described in CN 103 058 721 A belongs to the technical field of organic agricultural product production, and particularly provides a method for planting organic strawberry by a micro-ecological technology and a preparation of a used micro-ecological bacteria liquid. In particular, the micro-ecological bacteria liquid is obtained by preparing a plurality of stock solutions. The components of the stock solutions comprise a photosynthetic bacterium stock solution, a *Beauveria bassiana* stock solution, trichoderma culture, pathogenic fungus protein and a biological organic bacterial fertilizer (nutritive water). The bacteria liquid can be used as a medicament for sterilizing and killing worms and also can be used as an organic fertilizer for using. The high-quality organic strawberry which meets the requirement of OFDC (Organic Food Development Center) standard can be produced by only spraying the bacterial liquid regularly. By adjusting and controlling a micro-ecological environment of microorganism, an effective microbial community in a microbial community in a plant body after spraying the micro-ecological bacteria liquid can be changed into a dominant bacterial community, so that effects such as nutrition competition of the dominant bacterial community and the like can be fully played, the number of harmful bacterial communities is controlled below an economic harm level, and a maladjusted microecosystem can be restored into balance, so that the best economic, ecological and social benefits are obtained.

The principal objective of study of Tuan Anh Pham et al. "Production of Blastospore of Entomopathogenic Beauveria bassiana in a Submerged Batch Culture" (2009) was to determine the optimal liquid culture conditions in shake flasks for maximal sporulation of *Beauveria bassiana.* The optimal initial pH for the spore production of *B. bassiana* using Potato Dextrose Broth was 5.2. The screening in shake flasks of carbon and nitrogen sources resulted in the identification of an optimal medium based on 3% sucrose and 1% casamino acid, with a C:N ratio of 22:4. Using this medium, a production level of 5.65 × 10⁷ spores per ml was obtained after 5 days of culture. Using 3% corn meal, 2% corn step powder, and 2% rice bran, the maximum spore concentration of 8.54×10⁸/ml was achieved 8 days after inoculation at 25°C in a rotary shaking incubator operated at 200 rpm. This represents a yield gain of approximately 2.89 times that of pre-optimization. This document does not disclose egg as N source, nor increased life time of the fungi.

Ravneel Kumar et al in study "Growth rate in media and pathogenicity of two species of entomopathogens found in Fiji" describe two species of entomopathogenic fungi, *Beauveria bassiana* and *Metarhizium anisopliae* which were collected in the field on larvae of the Black and Yellow Mud Dauber *Sceliphron caementarium* in the Instructional Agricultural Farm Complex of the Fiji National University's College of Agriculture, Fisheries and Forestry. These fungi were cultured using different nutrient media to determine the type best suited for isolation and mass production. It was identified that both species responded well to growth in Egg Glucose Agar while a slower rate of mycelial formation occurred in Potato Dextrose Agar over a 7-day period under a photoperiod of 16 hours light and 8 hours of darkness at a relative humidity of 85%. Pathogenicity tests on *Helicoverpa armigera* larvae indicated that the isolate of *Metarhizium anisopliae* was more virulent than the strain of *Beauveria bassiana* with an LT50 time of 4 and 6 days respectively. *B. bassiana* has not previously been collected in the country while significant gaps still remain in the understanding of the potential for use of bio-control agents in agriculture locally.

The non-patent document "Mass Production of Fungal Entomopathogenes" (Jaronski et al., 2000) is a review discussing different solid substrates used for production of entomopathogenic fungi: *Beauveria bassiana* and *Metarhizium anisopliae* including cereals flour, bran and cakes. This document does not disclose whole egg as N source, nor increased life time of the fungi.

The non-patent document "Photoprotection of Beauveria bassiana: testing simple formulations for control of the coffee berry borer" (Steven Edgington et al, 2000) discloses that egg albumen and milk powder efficiently protect *B. bassiana* against UV irradiation. This document does not disclose whole egg, nor increased life time of the fungi.

The non-patent document "The effects of natural substrates on the sporulation and viability of conidia and blastospores of Metarhizium anisopliae" (Siamak Roshandel et al., 2016) discloses the influence of different media on sporulation and storage of *Metarhizium anisoplia* in liquid and solid media. The media comprising whole eggs were not disclosed.

The non-patent document "Production and shelf life evaluation of three different formulations of Beauveria bassiana in terms of multimetal removal" (Gola Deepak et al., 2019) discloses storage stable myco-tablets 8 mm diameter and 3 mm width comprising *Beaveria bassiana* and rice fluor suitable for the application in water. The mixture of rice flour with egg as nutrient source was not disclosed.

As biological protection of plants represents an environmentally friendly solution of protection from pests and demand for its use keeps increasing, it is desirable to look for new, progressive solutions how to ensure the longest possible protection period with the highest possible level of protection of plants from pests at the lowest possible cost, while ensuring biological protection from pests that are not affected by traditional methods of application.

### Nature of Invention

The aforementioned problems are resolved by the biological preparation for plant protection against pests, which contains a carrier of biologically active organism, and the biologically active organism, essentially based on the carrier composed of homogeneous blend of dry component and eggs. The dry component is a blend of middlings and flour, with preferable ratio from 2:1 to 1:2 or only middlings or only flour. The amount of eggs relative to the amount of dry component is between 60 % and 84 % by weight. The biologically active organism are entomopathogenic fungi, whose mycelium has grown over and inside the carrier. Mycelium of this fungus produces spores on the surface of the carrier. Middlings, flour and eggs are commonly available raw materials both environmentally friendly and affordable. The carrier is in the shape of a spatial arrangements with diameter at least 0.5 cm. The carrier is intended for application in the terrain by freely throwing.

The biological preparation for plant protection is designed so that it protects the mycelium of the entomopathogenic fungus from adverse conditions, such as exposure to UV radiation or to drought conditions.

The solution according to the present invention represents a carrier of preferably spherical shape with mycelium of an entomopathogenic fungus located on the surface and within the volume of the carrier. The carrier can attain other spatial arrangements than spherical. For instance, it can be shaped as cylinder, cube or cuboid, etc. In adverse conditions, not favorable to the entomopathogenic fungus, such as drought or exposure to UV radiation, the mycelium of the entomopathogenic fungus on the carrier's surface is inactivated. Solution according to this invention maintains appropriate conditions for the entomopathogenic fungus inside the carrier. If external conditions reappear, subsequently, that are favorable to the entomopathogenic fungus, e.g. after reduction in UV radiation (seasonal change, relocation to shade, beginning of humid period, end of drought, etc.), the entomopathogenic fungus will re-grow from inside of the carrier to the surface and will again release spores to the environment, which cause death of pests, selectively depending on the strain and species of the entomopathogenic fungus.

The favorable conditions for mycelium of the entomopathogenic fungus inhabiting the carrier are safeguarded by the carrier being produced from energetically nutritious ingredients and, at the same time, being able to absorb humidity from soil and/or from its environment. This way, the entomopathogenic fungus can live and sporulate (grow spores) even in summer months. The carrier can preferably obtain humidity from the environment (including soil) near the soil surface. This way, the biologically active entomopathogenic fungus can be maintained in its natural environment for its entire vegetation period (around 6 months), which represents a significantly extended effectiveness of the solution according to the present invention, either compared to superficial spraying of aqueous solution with entomopathogenic fungal spores or compared to a hard carrier that only contains wheat grains.

Middlings and flour are natural materials supporting fungal growth. The preparation of carrier can involve pure middlings only or pure flour only. The middlings are convenient to use as the price is lower than that of flour. The flour is convenient to use as it is milled to a finer fraction than the middlings. The most preferable embodiment, for both economical, technological and production reasons, is the use of mixture with middlings and flour.

In the manufacture of the carrier, cereal meal, corn meal, legume meal or mixtures thereof can be used and flour used can be cereal flour, corn flour or mixtures thereof.

It is possible to use barley meal, wheat meal, maize meal, oat meal or rye meal from cereal meal. The flour can be used smooth, but it can also be semi-coarse or coarse.

In the case, the mixture of dry component and eggs is too dry, it is possible to add water to the mixture in such an amount that the mixed mixture is homogeneous.

It is preferable if the carrier is of spherical or cylindrical shape with diameter at least 0.5 cm. Such shape provides the entomopathogenic fungus with favorable conditions inside the carrier. The higher the diameter of the carrier, the higher the amount of suitable material it contains and the more suitable its environment (humidity) for survival and growth of mycelium of entomopathogenic fungi.

The carrier can preferably also contain substances supporting longer lifetime of the entomopathogenic fungi where the supporting substances are growth media with their content in the carrier up to 5 % by weight. Such growth media include Sabouraud Dextrose Agar, Fetal Bovine Serum, Grace's Insect Medium and other.

The carrier may preferably include pheromones or attractants, which attract the pests, thereby increasing the effectiveness of the preparation for plant protection according to this invention.

A method for preparing the biological preparation for plant protection according to the present invention includes the following steps:
- the middlings and/or flour are sterilized at temperature of 110 °C to 130 °C, a pressure of 210 kPa for 10 to 30 minutes;
- after the middlings and/or flour have cooled down, eggs are added and the mixture is stirred until homogeneous;
- the stirred mixture is shaped into the carrier, preferably shaped as sphere or cylinder or cube or cuboid;
- the shaped carrier is dried in a desiccator for 2 hrs to 4 hrs at a temperature from 40 °C to 60 °C;
- the dried carrier is sterilized at a temperature from 110 °C to 130 °C, pressure of 210 kPa for 10 to 30 minutes;
- the carrier is left to cool down to room temperature;
- the cooled carrier is poured over by the suspension of entomopathogenic fungi, the concentration of spores per 1 ml is from 10⁵ to 10¹⁰, preferably 10⁸, wherein the suspension is prepared by mixing water and spores of entomopathogenic fungi;
- after pouring the suspension of entomopathogenic fungi over the carrier, the fungi are left to cultivate at temperature from 15 °C to 25 °C (ideally 22 °C ±2 °C) for 10 to 20 days in a sterile room, at an ambient humidity of at least 50 %,
- fungi are cultivated for 6-7 days in total darkness, which induces formation of the fungal mycelium, which colonizes the carrier;
- once the fungal mycelium is overgrown over the entire surface of the carrier, the light is switched on for 6-7 days, to initiate spore formation on the superficial mycelium.

It is preferable if the individual steps up to the step of pouring the carrier over with a suspension of entomopathogenic fungi are carried out in a sterile environment, e. g. under a UVC lamp, which ensures the sterility of the environment.

The preparation for plant protection according to present invention is applied in the terrain by freely throwing it out on a clearing and/or is placed in a natural attractant (in the trapping bark) or in close proximity to a natural attractant and/or is placed in a pit in the soil together with a pheromone or attractant and/or or is placed in a technical apparatus where the target pest will be attracted by the pheromone or attractant and at the same time the preparation for plant protection is protected from direct sunlight.

The entomopathogenic fungus carrier according to the present invention for use against insect pests is preferably used when the pests are beetles (e. g. large pine weevil, European spruce bark beetle), whose larvae live under the bark and beetles live scattered in the growth.

Many entomopathogenic fungi can be placed in the carrier according to the present invention, e. g. from the genera *Beauveria* or *Metarhizium* or *Isaria.*

The advantages of the biological preparation for plant protection according to the present invention are the long survival of the fungus in the carrier, the simple application in the terrain and the affordable price of the final product. The carrier can be freely thrown out on a clearing, placed in the trapping bark, in a special trap (technical apparatus) or to seedlings, which e. g. large pine weevil (*Hylobius abietis*) actively seeks out and performs a mature or nutritious eat. The carrier is preferably placed e. g. into a trapping bark with dimensions of approx. 20 × 40 cm, which is made of fresh spruce bark and which attracts large pine weevil very well. The proposed carrier is covered with such a bark, the fungus on the carrier produces spores which attach to the large pine weevil imago, which was attracted by the scent (by primary attractants) of the bark, causing its infection. Thus, infected large pine weevil imago can be killed by an entomopathogenic fungus within one week, or it can transmit the infection when mated or in contact with another large pine weevil. This ensures a targeted and selective form of biological control of this pest.

The carrier according to the present invention can also be used as a substrate for other entomopathogenic fungi, e.g. *Metarhizium*, *Isaria*, etc. against forest or agricultural pests. Selection and attraction of target organisms (pests) is provided either by pheromones or attractants.

### Description of Figures

Fig. 1 The course of the average mortality of large pine weevil images and their overgrowth rate by the mycelium of the entomopathogenic fungus *Beauveria bassiana* during individual controls
Fig. 2 Total average mortality of large pine weevil images and their overgrowth rate by the mycelium of the entomopathogenic fungus *Beauveria bassiana* for all controls
Fig. 3 Total number of dead large pine weevil images after the completion of the experiment
Fig. 4 Total percentage of survival of large pine weevil images after the completion of the experiment
Fig. 5 Statistically significant difference in survival of large pine weevil images between "control" and "carrier"

### Examples of Embodiments

The invention is further illustrated by the following non-limiting examples of embodiments:

### Example 1

The biological preparation for plant protection according to the present invention contains an entomopathogenic fungus carrier which is overgrown with entomopathogenic fungal mycelium, i. e. on the surface and also inside and the mycelium produces spores. In this example of embodiment, an entomopathogenic fungus *Beauveria bassiana* is used which has a selective effect on killing the large pine weevil. The biological preparation for plant protection according to this example is specifically intended for the protection of plants against large pine weevil.

The carrier is a mixture containing 150g barley meal, 75g wheat flour, i. e. the amount of middlings and the amount of flour is in the ratio 2:1 and further contains 3 eggs of size M (the weight of one egg is 54-63 g.), i.e. 162 g up to 189 g eggs. The amount of eggs based on the amount of middlings and flour is from 72% by weight to 84% by weight. The flour used is smooth.

The carrier has a spherical shape with a diameter of 2.4 cm.

The method for producing a biological preparation for plant protection according to this example of embodiment is as follows:
First, we sterilize the flour and middlings in an autoclave at 121 °C and a pressure of 210 kPa for 20 minutes. Then the ingredients are left to cool down. We pour the cooled flour and middlings into a food processor and add eggs. We stir until the mixture is homogeneous (about 1 minute), when there is more mixture, it takes longer. The mixed mixture is then inserted into the dough discharge gun, where the outlet opening is 24 mm. Using this gun, we extrude a puddle of the mixture, which we then place in a rollball size of 24 mm, where the carriers are formed into a spherical shape. Rollballs and extruders have different sizes (diameters), so it is possible to make smaller but also larger carriers. The carriers produced in this way are then placed in a fruit dryer at 50°C for 3 hours. After removing from the dryer, we store the carriers in petri dishes (diameter of the dish 20 cm, height 3 cm), or in another container suitable for use in an autoclave. From 30 to 40 carriers can be placed in one 20x3 cm petri dish. Thus, the carriers are sterilized in an autoclave at 121 °C and a pressure of 210 kPa for 20 minutes. The carriers are then left to cool down to room temperature. We perform all the above-mentioned operations under a UVC lamp, which ensures the sterility of the surrounding environment. We prepare a suspension of entomopathogenic fungi. The concentration of spores in 1 ml is 10⁸. The number of spores is determined under a microscope. The cooled carriers are then poured over by the suspension of entomopathogenic fungi. One carrier contains about 4 ml of a suspension of entomopathogenic fungi, i. e. when there are 30 carriers in one dish, we pour about 120 ml of a suspension of entomopathogenic fungi. The carriers prepared in this way are then placed in a sterile room where the temperature is 22°C (± 2°C). Cultivation at 22°C takes 14 days. At ambient humidity min. 50% the fungi are cultivated. Carriers poured over with a suspension of entomopathogenic fungi are 6-7 days in total darkness, which induces formation of the fungal mycelium. Once the fungal mycelium is overgrown over the entire surface of the carrier, the light is switched on for 6-7 days to initiate spore formation on the superficial mycelium. The fungal mycelium starts to produce spores when illuminated. This moment is very important. The carrier is preferably overgrown when we touch the mycelium and a coating of spores remains on our fingers. After these 2 weeks, we obtain a biological preparation for plant protection according to the present invention comprising a carrier which is completely overgrown with mycelium of an entomopathogenic fungus capable of producing fungal spores. The product is ready for application in the terrain.

### Experiment in laboratory conditions

The experiment was performed in the laboratories of the National Forestry Center, Center of Forestry Protection Service in Banská Štiavnica with 360 large pine weevil images, and after two weeks of the experiment more than 90% of the images that came into contact with the entomopathogenic fungus were dead.

300 images were placed in petri dishes together with the biological preparation for plant protection according to the present invention in five different variants of 60 images each and 60 images were used as a control in the absence of the biological preparation for plant protection.

300 images were divided into five blocks of 60 imagps. Each block consisted of 10 petri dishes and each had 6 large pine weevil images. Biological preparations for plant protection according to the present invention were placed in each block with 60 images as follows:
- Block A: freshly produced biological preparations - not exposed to any negative effects.
- Block B: biological preparations placed for 120 hours under a UVC lamp, thus simulating solar radiation that kills the entomopathogenic fungus *Beauveria bassiana.*
- Block C: biological preparations placed in a freezer at the temperature of -18 ° C for 120 hours to simulate frost.
- Block D: Biological preparations placed to the images for 24 hours and then placed into a sterile petri dish (without *Beauveria bassiana* spores) to see if brief contact of the images with the biological preparation was sufficient.
- Block E: biological preparations that were infected with the entomopathogenic fungus *Beauveria bassiana* two months before the experiment and placed in a refrigerator at the temperature of 4°C.
- Block K: control images in the absence of biological preparation.

The degree of mortality and overgrowth of large pine weevil images by the mycelium of the entomopathogenic fungus *Beauveria bassiana* was assessed at each control as follows:
1- living individual
2- dead without overgrowth
3- dead overgrown to 1/3
4- dead overgrown to 2/3
5- dead overgrown over 2/3

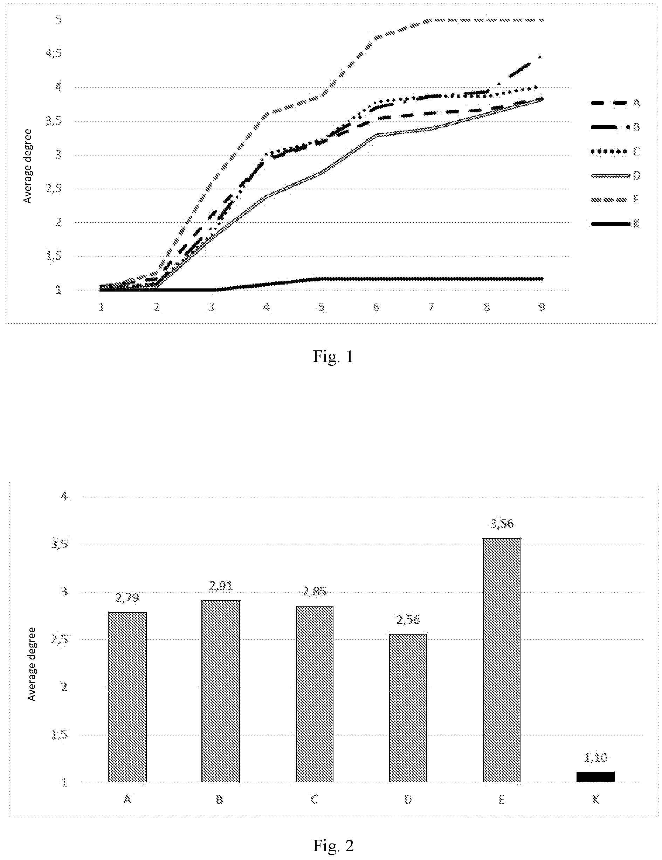

The results are shown in Fig. 1 and Fig. 2.

### Experiment in external insectariums

### Methodology

The experiment was performed in external insectariums of the National Forestry Center, Center of Forestry Protection Service in Banská Štiavnica in semi-natural conditions (natural alternation of temperature, light and humidity conditions, irrigation added artificially).

20 breeding cages were used for the experiment. A pot with a substrate suitable for planting conifers was placed in each breeding cage, where 4 spruce seedlings (*Picea abies*) were planted at a distance of 25x25 cm. A total of 80 seedlings (20x4) were planted in the experiment. A trapping bark made of fresh spruce bark measuring 10x20 cm was inserted in the middle between the seedlings, which was folded in half (10x10 cm, by lining inwards). The bark was laid and lightly pressed against the substrate. A biological preparation for plant protection according to this example of embodiment, which produced spores of the entomopathogenic fungus *Beauveria bassiana,* was placed in the middle of the cages (10 pieces) in the middle of the bark. In this way, we simulated the use of this preparation in trapping barks, which are used to monitor the large pine weevil in terrain conditions. The other half of the cages were free of the biological preparation for plant protection in the trapping bark and served as a control. The seedlings and substrate were watered at regular intervals.

At the beginning of the experiment, 4 spruce large pine weevil images were placed in each cage. An additional 4 spruce hardwood images were inserted at each measurement (every 14 days). A total of 400 images were placed in all cages for the entire experiment (20 images in each cage). At the end of the experiment, we searched for all the images that were placed in the cages and evaluated whether they were alive or overgrown with an entomopathogenic fungus. The substrate from each pot separately was carefully examined to find dead images.

All dead images in cages in the presence of the biological preparation were also overgrown with the entomopathogenic fungus *B. bassiana.* The dead images in the control cages were not overgrown with the fungus and died of other causes.

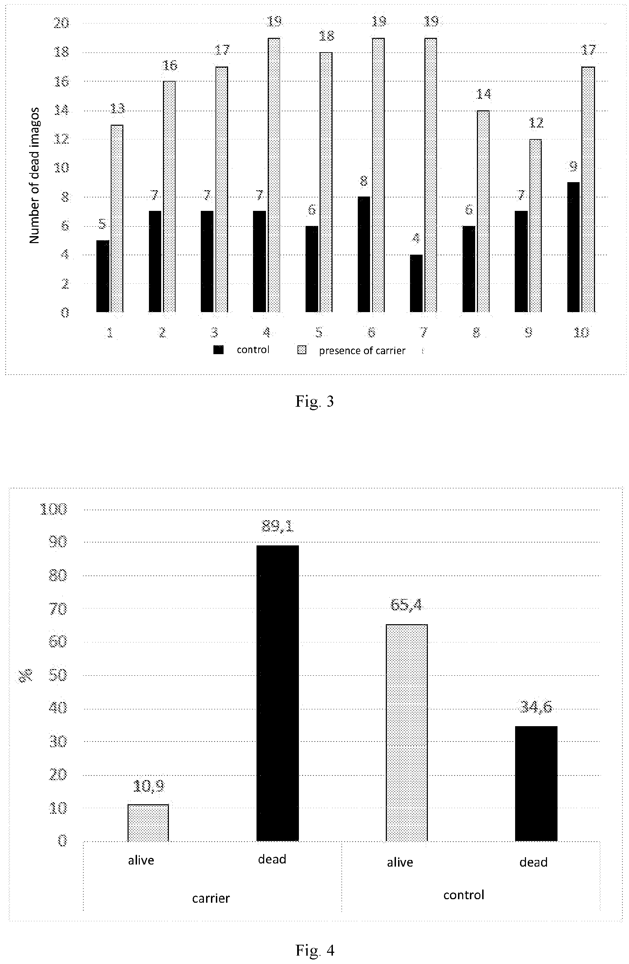

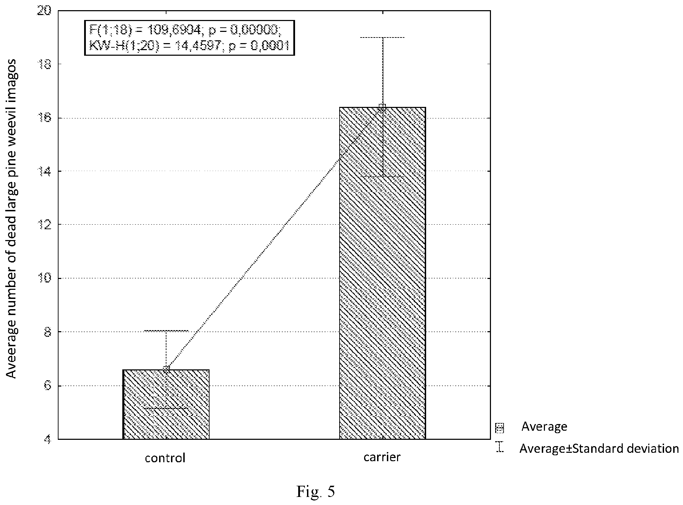

The results are shown in Fig. 3, Fig. 4 and Fig.5.

### Example 2

Example 2 is identical to Example 1, except that the biological preparation is cylindrical and the diameter of the cylinder is from 0.5 cm.

### Example 3

Example 3 is identical to Example 1, except that water was added to the mixture of middlings, flour and eggs, since the mixed mixture was not sufficiently homogeneous. Sufficient water is added to make the mixture homogeneous after mixing.

### Example 4

Example 4 is identical to Example 1 except that corn meal is used.

### Example 5

Example 5 is identical to Example 1, except that wheat meal without flour is used.

### Example 6

Example 6 is identical to Example 1 except that coarse oatmeal is used without the use of middlings.

### Example 7

Example 7 is identical to Example 1, except that the carrier also contains substances supporting longer lifetime of the entomopathogenic fungi, and these supporting substances are growth media, wherein the content of growth media is 3% by weight.

### Example 8

Example 8 is identical to Example 1 except that a specific pheromone is used to attract the target pest species instead of the trapping bark.

### Example 9

Example 9 is identical to Example 1, except that an artificial or natural attractant is used to attract the target pest species instead of a trapping bark.

### Example 10

Example 10 is identical to Example 1 except that a specific pheromone or an artificial or natural attractant which is part of a biological preparation for plant protection is used instead of the trapping bark.

### Example 11

Example 11 is identical to Example 1, except that a specific pheromone and/or an artificial or natural attractant is added to the trapping bark in addition to the preparation for plant protection to increase attractiveness.

## Claims

1. A biological preparation suitable for use in biological plant protection against pests containing a carrier of a biologically active organism and a biologically active organism, wherein the carrier of the biologically active organism contains a mixture of dry component and eggs **characterized in that** the dry component is a blend of middlings and flour or only middlings or only flour and the amount of eggs relative to the amount of dry component is from 60% by weight to 84% by weight and the biologically active organism are entomopathogenic fungi, whose mycelium is located on the surface and inside the carrier and is capable of producing spores, the carrier is in the shape of a spatial arrangements with diameter at least 0.5 cm; intended for application in the terrain by freely throwing.

2. The biological preparation for plant protection according to claim 1, **characterized in that** the middlings is cereal meal, corn meal, legume meal or mixtures thereof and the flour is cereal flour, corn flour or a mixture thereof.

3. The biological preparation for plant protection according to any one of claims 1 and 2, **characterized in that** it further comprises water in such an amount that after mixing the preparation is homogeneous.

4. The biological preparation for plant protection according to any one of claims 1 to 3, **characterized in that** the spatial arrangement is a sphere, a cylinder, a cube, a cuboid.

5. The biological preparation for plant protection according to any one of claims 1 to 4 **characterized in that** the carrier further contains Sabouraud Dextrose Agar or Fetal Bovine Serumor Grace's Insect Medium in the amount up to 5% by weight.

6. The biological preparation for plant protection according to any one of claims 1 to 5, **characterized in that** it further contains pheromones or attractants.

7. A method for preparing the biological preparation for plant protection according any of claims 1-6 including the following steps:
- the middlings and/or flour are sterilized at a temperature of 110 °C to 130 °C, a pressure of 210 kPa for 10 minutes to 30 minutes,
- after the middlings and/or flour have cooled down, eggs are added and the mixture is stirred until homogeneous,
- the carrier is shaped from the mixed mixture into a spatial arrangement,
- the shaped carrier is dried in a desiccator for 2 hours to 4 hours at a temperature from 40 ° C to 60 ° C
- the dried carrier is sterilized at a temperature from 110 °C to 130 °C, a pressure of 210 kPa for 10 minutes to 30 minutes,
- the carrier is left to cool down to room temperature,
- the cooled carrier is poured over by the suspension of entomopathogenic fungi, the concentration of spores per 1 ml is from 10⁵ to 10¹⁰, preferably 10⁸, wherein the suspension of entomopathogenic fungi is prepared by mixing water and spores of entomopathogenic fungi,
- after pouring the suspension of entomopathogenic fungi over the carrier, the fungi are left to cultivate at a temperature from 15 °C to 25 °C for 10 days to 20 days in a sterile room, at an ambient humidity of at least 50%,
- fungi are cultivated for 6-7 days in total darkness, which induces formation of the fungal mycelium, which colonizes the carrier,
- once the fungal mycelium is overgrown over the entire surface of the carrier, the light is switched on for 6-7 days, to initiate spore formation on the superficial mycelium.

8. The method of using the biological preparation for plant protection as defined in claims 1-6 or as produced by the method of claim 7, **characterized in that** it is applied by freely throwing it out in the terrain and/or is placed in a natural attractant or in close proximity to a natural attractant, and/or is placed in a pit in the soil together with a pheromone or attractant and/or is placed in a technical apparatus where the target pest will be attracted by the pheromone or attractant and at the same time the preparation for plant protection is protected from direct sunlight.

## Patentansprüche

1. Biologisches Präparat, geeignet zur Verwendung im biologischen Pflanzenschutz gegen Schädlinge, enthaltend einen Träger eines biologisch aktiven Organismus und einen biologisch aktiven Organismus, wobei der Träger des biologisch aktiven Organismus eine Mischung aus einer Trockenkomponente und Eiern enthält, **dadurch gekennzeichnet, dass** die Trockenkomponente eine Mischung aus Schrot und Mehl oder nur Schrot oder nur Mehl ist und die Menge an Eiern bezogen auf die Menge der Trockenkomponente von 60 Gew.-% bis 84 Gew.-% beträgt und der biologisch aktive Organismus entomopathogene Pilze sind, deren Myzel sich auf der Oberfläche und im Inneren des Trägers befindet und zur Sporenbildung befähigt ist, wobei der Träger die Form einer räumlichen Gestaltung mit einem Durchmesser von mindestens 0,5 cm einnimmt; bestimmt zur Anwendung im Gelände durch freies Ausstreuen.

2. Biologisches Pflanzenschutzpräparat nach Anspruch 1, **dadurch gekennzeichnet, dass** das Schrot ein Getreideschrot, ein Maisschrot, ein Hülsenfruchtschrot oder Mischungen davon ist und das Mehl ein Getreidemehl, ein Maismehl oder eine Mischung davon ist.

3. Biologisches Pflanzenschutzpräparat nach einem der Ansprüche 1 und 2, **dadurch gekennzeichnet, dass** es zusätzlich Wasser in einer solchen Menge enthält, dass das Präparat nach dem Mischen homogen ist.

4. Biologisches Pflanzenschutzpräparat nach einem der Ansprüche 1 bis 3 **dadurch gekennzeichnet, dass** die räumliche Gestaltung eine Kugel, ein Zylinder, ein Würfel, ein Quader ist.

5. Biologisches Pflanzenschutzpräparat nach einem der Ansprüche 1 bis 4 **dadurch gekennzeichnet, dass** der Träger zusätzlich Sabouraud-Dextrose-Agar oder Fetales Rinderserum oder Graces Insektenmedium in einer Menge von bis zu 5 Gew.-% enthält.

6. Biologisches Pflanzenschutzpräparat nach einem der Ansprüche 1 bis 5 **dadurch gekennzeichnet, dass** es zusätzlich Pheromone oder Lockstoffe enthält.

7. Verfahren zur Herstellung eines biologischen Pflanzenschutzpräparats nach einem der Ansprüche 1-6, umfassend die folgenden Schritte:
- das Schrot und/oder das Mehl werden bei einer Temperatur von 110 °C bis 130 °C und einem Druck von 210 kPa über einen Zeitraum von 10 bis 30 Minuten sterilisiert,
- nach dem Abkühlen werden Eier zu dem Schrot und/oder dem Mehl zugegeben und die Mischung wird gerührt, bis die Mischung homogen ist,
- der Träger wird aus der gemischten Mischung zu einer räumlichen Gestaltung geformt,
- der geformte Träger wird in einem Trockner über einen Zeitraum von 2 bis 4 Stunden bei einer Temperatur von 40 °C bis 60 °C getrocknet,
- der getrocknete Träger wird bei einer Temperatur von 110 °C bis 130 °C und einem Druck von 210 kPa über einen Zeitraum von 10 bis 30 Minuten sterilisiert,
- der Träger wird auf Raumtemperatur abkühlen gelassen,
- der abgekühlte Träger wird mit einer Suspension entomopathogener Pilze übergossen, die Konzentration der Sporen pro 1 ml beträgt 10⁵ bis 10¹⁰, vorzugsweise 10⁸, wobei die Suspension entomopathogener Pilze durch Mischen von Wasser und Sporen entomopathogener Pilze hergestellt wird,
- nach dem Übergießen des Trägers mit der Suspension entomopathogener Pilze werden die Pilze bei einer Temperatur von 15 °C bis 25 °C über einen Zeitraum von 10 bis 20 Tage in einem sterilen Raum bei einer Umgebungsfeuchtigkeit von mindestens 50 % kultiviert,
- die Pilze werden 6-7 Tage lang in völliger Dunkelheit kultiviert, wobei sich das Myzel des Pilzes bildet, das den Träger besiedelt,
- sobald das Myzel des Pilzes über die gesamte Oberfläche des Trägers gewachsen ist, wird das Licht eingeschaltet, das 6-7 Tage lang leuchten gelassen wird, um die Sporenbildung auf dem oberflächlichen Myzel einzuleiten.

8. Verfahren zur Verwendung des biologischen Pflanzenschutzpräparats gemäß den Ansprüchen 1-6 oder hergestellt nach dem Verfahren gemäß Anspruch 7, **dadurch gekennzeichnet, dass** es durch freies Ausstreuen im Gelände ausgebracht wird und/oder in einen natürlichen Lockstoff oder in der unmittelbaren Nähe eines natürlichen Lockstoffs platziert wird und/oder zusammen mit einem Pheromon oder Lockstoff in eine Grube im Boden platziert wird und/oder in ein technisches Gerät untergebracht wird, wo der Zielschädling von dem Pheromon oder den Lockstoff angelockt wird und gleichzeitig das Pflanzenschutzpräparat vor direkter Sonneneinstrahlung geschützt wird.

## Revendications

1. Produit biologique adapté à l'utilisation pour la protection biologique de plantes contre des nuisibles contenant un support d'un organisme biologiquement actif et un organisme biologiquement actif, le support de l'organisme biologiquement actif comprenant un mélange d'un composant sec et d'oeufs **caractérisé en ce que** le composant sec est un mélange de grain concassé et de farine ou uniquement du grain concassé ou uniquement de la farine et la quantité d'oeufs est comprise entre 60 % en poids et 84 % en poids par rapport à la quantité de composant sec et l'organisme biologiquement actif sont des champignons entomopathogènes, dont le mycélium se trouve à la surface et à l'intérieur du support et est capable de produire des spores, le support se présente sous la forme d'une formation spatiale d'un diamètre d'au moins 0,5 cm ; destiné à être appliqué sur le terrain par diffusion libre.

2. Produit biologique pour la protection des plantes selon la revendication 1, **caractérisé en ce que** le grain concassé est constitué de céréales concassées, de maïs concassé, de légumineuses concassées ou leurs mélanges et la farine est la farine de céréales, la farine de maïs ou leur mélange.

3. Produit biologique pour la protection des plantes selon l'une quelconque des revendications 1 et 2, **caractérisé en ce qu'**il comprend en outre de l'eau en quantité telle que le produit soit homogène après mélange.

4. Produit biologique pour la protection des plantes selon l'une quelconque des revendications 1 à 3, **caractérisé en ce que** la formation spatiale est une sphère, un cylindre, un cube, un pavé droit.

5. Produit biologique pour la protection des plantes selon l'une quelconque des revendications 1 à 4, **caractérisé en ce que** le support comprend en outre de la Sabouraud Dextrose Agar ou du Fetal Bovine Sérum ou du Grace's Insect Medium en une quantité allant jusqu'à 5 % en poids.

6. Produit biologique pour la protection des plantes selon l'une quelconque des revendications 1 à 5, **caractérisé en ce qu'**il comprend en outre des phéromones ou des attractifs.

7. Méthode de préparation du produit biologique pour la protection des plantes selon l'une quelconque des revendications 1 à 6, contenant les étapes suivantes :
- le grain concassé et/ou la farine sont stérilisés à une température de 110 °C à 130 °C, une pression de 210 kPa pendant une durée de 10 minutes à 30 minutes,
- après refroidissement, les oeufs sont ajoutés au grain concassé et/ou à la farine et le mélange est agité jusqu'à ce que le mélange soit homogène,
- un support est façonné à partir du mélange pour avoir la forme d'une formation spatiale,
- le support façonné est séché dans le séchoir pendant une durée de 2 heures jusqu'à 4 heures à une température de 40 °C à 60 °C,
- le support séché est stérilisé à une température de 110 °C à 130 °C, une pression de 210 kPa pendant une durée de 10 minutes à 30 minutes,
- le support est laissé refroidir à température ambiante,
- le support refroidi est arrosé d'une suspension de champignons entomopathogènes, la concentration de spores dans 1 ml est de 10⁵ à 10¹⁰, avantageusement 10⁸, la suspension de champignons entomopathogènes étant préparée en mélangeant de l'eau et des spores de champignons entomopathogènes,
- après avoir arrosé le support d'une suspension de champignons entomopathogènes, les champignons peuvent être cultivés à une température de 15 °C à 25 °C pendant une période de 10 à 20 jours dans une pièce stérile, à une humidité ambiante d'au moins 50 %,
- les champignons sont cultivés pendant 6 à 7 jours dans l'obscurité totale, alors que le mycélium du champignon se forme qui colonise le support,
- lorsque le mycélium du champignon s'est développé sur toute la surface du support, la lumière est allumée et laissée allumée pendant 6 à 7 jours pour initier la formation de spores sur le mycélium de surface.

8. Méthode d'utilisation du produit biologique pour la protection des plantes tel que défini dans les revendications 1 à 6 ou tel que préparé par la méthode de la revendication 7, **caractérisé en ce qu'**il est appliqué sur le terrain par diffusion libre et/ou placé dans un attractif naturel ou à proximité immédiate d'un attractif naturel et/ou est placé dans un trou dans le sol avec la phéromone ou l'attractif et/ou placé dans un dispositif technique où l'organisme nuisible cible sera attiré par la phéromone ou l'attractif et en même temps, le produit pour la protection des plantes sera protégé des rayons directs du soleil.
